# EUROPÄISCHE PATENTSCHRIFT

(11) **EP 2 488 132 B1**
(45) Veröffentlichungstag und Bekanntmachung des Hinweises auf die Patenterteilung: **08.08.2018**
(21) Anmeldenummer: 10771692.0
(22) Anmeldetag: 11.10.2010
(51) Int. Cl.: A61F 5/00, A61F 5/441, A61F 2/00, A61F 5/44

(54) **EINRICHTUNG FÜR EINE DRUCKGEMINDERTE ABGABE VON DARMGASEN**
DEVICE FOR A PRESSURE-REDUCED DELIVERY OF INTESTINAL GASES
DISPOSITIF POUR UNE ÉMISSION À PRESSION RÉDUITE DE GAZ INTESTINAUX

(30) Priorität: 12.10.2009 AT 63309 U
(43) Veröffentlichungstag der Anmeldung: 22.08.2012
(73) Patentinhaber: Trimmel, Leopold, 3202 Hofstetten-Grünau (AT)
(72) Erfinder: Trimmel, Leopold, 3202 Hofstetten-Grünau (AT)
(74) Vertreter: Müllner, Martin
(86) Internationale Anmeldenummer: PCT/EP2010/065211
(87) Internationale Veröffentlichungsnummer: WO 2011/045278

(56) Entgegenhaltungen:
- WO-A2-2007/130953
- WO-A2-2009/060437
- DE-A1- 2 747 245
- DE-C- 637 552
- DE-U1-202006 005 951
- US-A- 1 610 947

## Beschreibung

### Technisches Gebiet

Die vorliegende Erfindung betrifft eine wegwerfbare Einrichtung zum einmaligen Gebrauch für eine druckgeminderte Abgabe von Darmgasen, die einen im Analkanal positionierbaren länglichen Körper mit einem wasser- und gasdichten Zylinder aus flexiblem Material aufweist.

### Stand der Technik

Derartige Einrichtungen sind z.B. aus der DE 7015484 U bekannt. Hier ist der Zylinder eine flexible, aus Kunststoff bestehende Röhre mit mindestens zwei seitlich angeordneten Langlöchern, die von glockenförmigen Lippen überdeckt sind.

Aus der EP 253130 A ist eine eingangs beschriebene Einrichtung bekannt, wobei der Zylinder in seiner Wandung Löcher aufweist und der Innenraum des Zylinders zum großen Teil als Sammelbehälter für die Darmgase dient, wobei der Zylinder ein inneres Röhrchen zum Ablassen der Gase aufweist.

Andererseits sind Vorrichtungen zum Verschließen der Darmöffnung bekannt:

Aus der EP 188376 A ist eine wegwerfbare Vorrichtung zum Verschluss einer Darmöffnung bekannt, wobei ein elastischer Körper vor dem Einsetzen durch ein Material, das auf Wärme und Feuchtigkeit empfindlich ist, in einem zusammengedrückten Zustand gehalten ist, sodass er sich nach dem Einsetzen unter der Wirkung seiner Elastizität ausdehnt und die Öffnung verschließt.

Nachteilig an den oben beschriebenen Einrichtungen zur druckgeminderten Abgabe von Darmgasen ist, dass es sich stets um Kunststoffröhren handelt, die geringen Tragekomfort bieten und händisch entfernt werden müssen, für möglichst langes Tragen konzipiert sind und immer die Problematik der Verstopfung der Gaseintrittslöcher durch Kot besteht.

Bei der Vorrichtung gemäß EP 188376 A handelt es sich zwar um eine wegwerfbare Vorrichtung, die aber wie ein Stöpsel wirkt und den Ausfluss von Flüssigkeiten und Gasen verhindern soll.

Aus der WO 2009/060437 A1 ist eine Einrichtung gemäß dem Oberbegriff des Anspruchs 1 bekannt.

### Darstellung der Erfindung

Aufgabe der vorliegenden Erfindung ist es, all diese Nachteile zu beseitigen und eine wegwerfbare Einrichtung der eingangs genannten Art zu schaffen, die für den Einmalgebrauch geeignet ist und den Komfort beim Tragen und beim Applizieren stark erhöht.

Erfindungsgemäß wird diese Aufgabe durch die Merkmale des kennzeichnenden Teils des Anspruchs 1 gelöst.

Insbesondere ist die Spitze bei der vorliegenden Erfindung von großer Bedeutung: sie ist durch Natur- und/oder Kunstfasern gebildet und hat eine mehrteilige Ausgestaltung durch eine Mehrzahl von um die Längsachse des Zylinders angeordneten Flügelblättern oder durch zusammengedrückte, schlaufenartig angeordnete Blätter. Dadurch ist das Risiko der Verstopfung durch Kot verringert und beim Einströmen der Gase in das Innere des Zylinders findet eine erste Druckreduktion statt.

Das Material im Inneren des Zylinders reduziert den Druck weiter und es kann auch parfümiert oder aus geruchneutralisierendem Material sein, sodass der störende Geruch der durchströmenden Gase vermindert werden kann. Das Zylinderinnere ist vorzugsweise aus biologisch leicht abbaubaren Materialien, die mäßig saugfähig sind (und nicht stark aufquellen), gefertigt.

Vor allem beim Einführen der Einrichtung in den Analkanal ist die Verletzungsgefahr hoch. Daher ist vorgesehen, dass die Spitze feucht ist.

Um die erfindungsgemäße Einrichtung insbesondere auch beim Schwimmen verwenden zu können, ist bevorzugt vorgesehen, dass der Körper zumindest ein Einwegventil bzw. eine Membran, vorzugsweise am in Funktionsstellung schließmuskelseitigen Ende, aufweist, sodass ein Eindringen von Gasen und Flüssigkeiten von außen in den Analkanal verhindert wird. Ebenso könnte es sonst nach dem Bücken und anderen Körperhaltungen zu einem Ansaugen von Luft kommen. Das Eindringen in den Analkanal kann durch ein besagtes Ventil bzw. eine besagte Membran verhindert werden.

Um die Positionierung und das Fixieren des Körpers in geeigneter Stellung zu gewährleisten, ist es günstig, wenn der Körper an seinem in Funktionsstellung schließmuskelseitigen Ende einen Anschlag besitzt, der in Funktionsstellung außerhalb des Schließmuskels positioniert ist und in den das Einwegventil bzw. die Membran integriert ist.

Dieser Anschlag kann durch eine Vergrößerung des Durchmessers des Zylinders am schließmuskelseitigen Ende bewerkstelligt werden.

Da Menschen individuell gebaut sind, erweist es sich als vorteilhaft, wenn an dem Zylinder außen weiches Material angeformt ist, um ein Anpassen an die individuelle Anatomie eines beliebigen Analkanals zu ermöglichen. Die äußere Form des Körpers kann z.B. rundlich, oval, konisch, zylindrisch, eingebuchtet, unregelmäßig sein, ein Relief aufweisen oder abstehende, z.B. flügelartige Teile besitzen.

Es erweist sich als sehr vorteilhaft, wenn der gesamte Körper außen befeuchtet bzw. mit einem Gleitmittel umgeben ist, weil dadurch einerseits das Einführen erleichtert wird und außerdem in Funktionsstellung die den Körper umschließenden Schleimhäute feucht bleiben.

Diese Gleit- bzw. Befeuchtungsmittel können z.B. Lotionen, Fette wie Vaseline, Öle oder Emulsionen sein, wobei diese auch Heil- und Pflegemittel sein können. Mit dem Auftragen eines solchen Mittels können auch medizinische Anwendungen, wie das Applizieren einer Hämorrhoidensalbe, unterstützt werden.

Da zu Gunsten des Tragekomforts die gesamte Einrichtung biegsam und weich ist, kann bei einer bevorzugten Ausführungsform der gesamte Körper spiralförmig verdreht gepresst sein. Auf diese Art und Weise wird eine Stabilisierung in Längsrichtung, insbesondere wenn Fasermaterial für das Äußere des Körpers verwendet wird, erreicht und das Applizieren ohne Hilfseinrichtung erleichtert.

An und für sich entfernt sich die Einrichtung bei jedem Stuhlgang von selbst. Es kann jedoch vorkommen, dass beim Einführen ein Handhabungsfehler passiert oder es durch unglückliche Umstände zu einer Fehlposition des Körpers kommt. Um die erfindungsgemäße Einrichtung problemlos entfernen zu können, hat der Körper am in Funktionsstellung schließmuskelseitigen Ende ein Band zum Entfernen der Einrichtung aus dem Körper angeformt.

Die Einrichtung für eine druckgeminderte Abgabe von Darmgasen kann händisch in Funktionsstellung im Analkanal gebracht werden.

Vorteilhaft ist eine Applikationshilfe, die aus einem Stift mit angeformter Verschiebevorrichtung besteht, wobei der Stift in der Längsachse des Zylinders positionierbar ist.

Eine andere vorteilhafte Applikationshilfe stellt ein offener Zylinder mit angeformter Verschiebevorrichtung dar, wobei der offene Zylinder dieselbe Längsachse wie der Körper selbst aufweist und in Funktionsstellung der offene Zylinder den Körper so umfasst, dass zumindest die Spitze frei bleibt.

### Kurze Beschreibung der Zeichnungen

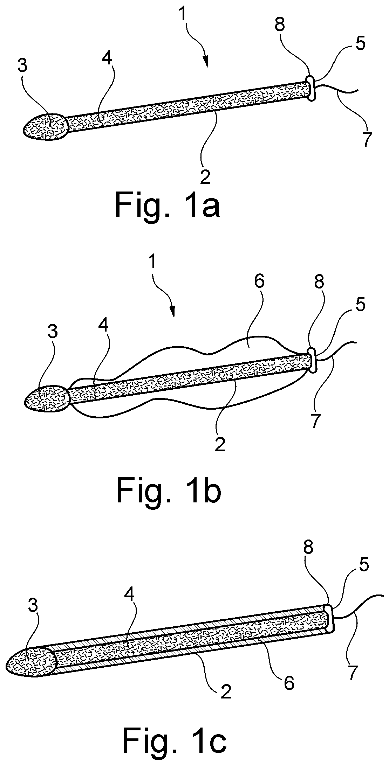

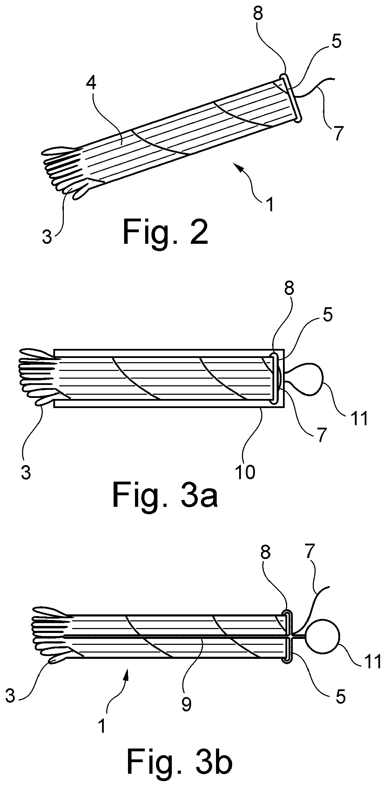

Eine bevorzugte Ausführungsform des erfindungsgemäßen Körpers zeigt Fig. 2; Fig. 3a und 3b zeigen zugehörige Applikationshilfen. Fig. 1a-1c zeigen weitere, nicht-erfindungsgemäßen Einrichtungen.

### Bester Weg zur Ausführung der Erfindung

Fig. 1a zeigt einen nicht-erfindungsgemäßen Körper 1, der einen flexiblen, wasser- und gasdichten Zylinder 2 aufweist. An dem Ende, das in Funktionsstellung sich weiter innen im menschlichen Körper befindet, ist eine feuchte Spitze 3 angeformt. Diese Spitze 3 ist aus weichem Material und nimmt vor dem Einführen des Körpers 1 in den menschlichen Körper an seiner breitesten Stelle eine größere Breite als der Zylinder 2 ein. Die Spitze 3 ist leicht zusammendrückbar. Das Zylinderinnere 4 ist mit weichem, großporigem Material gefüllt, im Gegensatz zu vielen herkömmlichen Einrichtungen zur druckgeminderten Abgabe von Darmgasen. Am in Funktionsstellung schließmuskelseitigen Ende ist ein Ventil 5 in einem Anschlag 8 integriert. Mit einem Band 7 kann der Körper 1 leicht aus dem Analkanal entfernt werden.
In Fig. 1b ist ein Körper 1 der Ausführung gemäß Fig. 1a dargestellt, wobei an den Zylinder 2 außen noch weiches Material 6 flügelartig angeformt ist.
In Fig. 1c ist das am Zylinder 2 außen angeformte Material 6 eine Art Polsterung in Form eines Außenzylinders.
In Fig. 2 ist eine erfindungsgemäße Ausführungsform, bei der der gesamte Körper 1 spiralförmig verdreht ist, dargestellt. Diese Form wird stärker bevorzugt verwendet, wenn der Zylinder 2 als dünnwandiger Schlauch mit großporigem Inneren ausgeführt ist. Durch das Verdrehen kann zusätzliche Formstabilisierung in Richtung der Längsachse des Körpers erreicht werden. Außerdem ist bei dieser gezeigten Ausführungsform die Spitze 3 mehrteilig bzw. mit einer vielfach gekrümmten Oberfläche ausgebildet.
Fig. 3a zeigt die Ausführungsform des Körpers nach Fig. 2 in einer Applikationshilfe, die aus einem offenen Zylinder 10 mit Verschiebevorrichtung 11 besteht. Nach Einsetzen des Körpers 1 mit der Applikationshilfe in den Analkanal wird die Applikationshilfe entfernt.
In Fig. 3b besteht die Applikationshilfe aus einem Stift 9, der in der Längsachse des Körpers 1 positioniert wird und eine Verschiebevorrichtung 11 trägt.

## Patentansprüche

1. Wegwerfbare Einrichtung zum einmaligen Gebrauch für eine druckgeminderte Abgabe von Darmgasen, die einen im Analkanal positionierbaren länglichen Körper (1) mit einem wasser- und gasdichten Zylinder (2) aus flexiblem Material aufweist, wobei das in Funktionsstellung dem schließmuskelseitigen Ende des Körpers (1) gegenüberliegende Ende eine Spitze (3) aus weichem Material ein- oder mehrteilig ausgebildet ist, diese Spitze (3) an seiner breitesten Stelle eine größere Breite als der Zylinder (2) aufweist, diese Spitze (3) feucht ist und das Zylinderinnere (4) aus weichem, großporigem, filterartigem Material besteht, **dadurch gekennzeichnet, dass** die Spitze (3) durch Natur- und/oder Kunstfasern gebildet ist, und eine vielfach gekrümmte Oberfläche aufweist und mehrteilig durch eine Mehrzahl von um die Längsachse des Zylinders (2) angeordneten Flügelblättern, die gegebenenfalls zusammengedrückt oder schlaufenartig angeordnet sind, ausgebildet ist.

2. Einrichtung nach Anspruch 1, **dadurch gekennzeichnet, dass** der Körper (1) zumindest ein Einwegventil (5) bzw. eine Membran aufweist, sodass ein Eindringen von Gasen und Flüssigkeiten von außen in den Analkanal verhindert wird.

3. Einrichtung nach Anspruch 2, **dadurch gekennzeichnet, dass** das Einwegventil (5) bzw. die Membran am in Funktionsstellung schließmuskelseitigen Ende angeordnet ist.

4. Einrichtung nach Anspruch 3, **dadurch gekennzeichnet, dass** der Körper (1) an seinem in Funktionsstellung schließmuskelseitigen Ende einen Anschlag (8) besitzt, der in Funktionsstellung außerhalb des Schließmuskels positioniert ist und in dem das Einwegventil (5) bzw. die Membran integriert ist.

5. Einrichtung nach einem der Ansprüche 1 bis 4, **dadurch gekennzeichnet, dass** an dem Zylinder (2) außen weiches Material (6) angeformt ist, um ein Anpassen an die individuelle Anatomie eines beliebigen Analkanals zu ermöglichen.

6. Einrichtung nach Anspruch 5, **dadurch gekennzeichnet, dass** der gesamte Körper (1) spiralförmig verdreht gepresst ist.

7. Einrichtung nach einem der Ansprüche 1 bis 6, **dadurch gekennzeichnet, dass** der Körper (1) am in Funktionsstellung schließmuskelseitigen Ende ein Band (7) zum Entfernen der Einrichtung aus dem Körper angeformt hat.

8. Einrichtung nach einem der Ansprüche 1 bis 7 mit einer Applikationshilfe, **dadurch gekennzeichnet, dass** die Applikationshilfe aus einem Stift (9) mit angeformter Verschiebevorrichtung (11) besteht, wobei der Stift (9) in der Längsachse des Zylinders (2) positionierbar ist.

9. Einrichtung nach einem der Ansprüche 1 bis 7 mit einer Applikationshilfe, **dadurch gekennzeichnet, dass** die Applikationshilfe aus einem offenen Zylinder (10) mit angeformter Verschiebevorrichtung (11) besteht, wobei der offene Zylinder (10) dieselbe Längsachse wie der Körper (1) selbst aufweist, und dass in Funktionsstellung der offene Zylinder (10) den Körper (1) so umfasst, dass zumindest die Spitze (3) frei bleibt.

## Claims

1. Disposable device for single use for pressure-reduced delivery of intestinal gases including an elongated body (1) positionable in the anal canal having a watertight and gas-tight cylinder (2) made of flexible material wherein the end opposing the sphincter side end of the body (1) in functional position has formed a tip (3) of one or more parts made of soft material, this tip (3) including at its widest location a larger width than the cylinder (2), this tip (3) being wet, and the interior (4) of the cylinder consisting of soft, large pored, filter-like material, **characterized in that** the tip (3) is formed by nature and/or synthetic fibres and includes a multiply curved surface and is formed by a plurality of blades arranged in several parts around the longitudinal axis of the cylinder (2), optionally compressed or arranged in a loop-like manner.

2. Device according to Claim 1, **characterized in that** the body (1) includes at least one one-way valve (5) or a membrane, respectively, so as to prevent the penetration of gases and liquids from the outside into the anal canal.

3. Device according to Claim 2, **characterized in that** the one-way valve (5) or the membrane, respectively, are arranged in functional position at the sphincter side end.

4. Device according to Claim 3, **characterized in that** the body (1) has an abutment (8) in functional position at its sphincter side end that in functional position is positioned outside of the sphincter and in which the one-way valve (5) or the membrane, respectively, is integrated.

5. Device according to any one of claims 1 to 4, **characterized in that** a soft material is formed to the cylinder (2) at the outside to allow for a fitting to the individual anatomy of any anal canal.

6. Device according to Claim 5, **characterized in that** the entire body (1) is pressed in a helically twisted manner.

7. Device according to any one of claims 1 to 6, **characterized in that** the body (1) has integrally formed a ribbon (7) in functional position on the sphincter side end for removing the device from the body.

8. Device according to any one of claims 1 to 7 with an application aid, **characterized in that** the application aid consists of a pin (9) having an integrally formed displacement device (11) wherein the pin (9) is positionable in the longitudinal axis of the cylinder (2).

9. Device according to one of claims 1 to 7 with an application aid, **characterized in that** the application aid consists of an open cylinder (10) having an integrally formed displacement device (11) wherein the open cylinder (10) includes the same longitudinal axis as the body (1) itself, and that in functional position the open cylinder (10) encloses the body (1) so that at least the tip (3) remains free.

## Revendications

1. Dispositif jetable à usage unique qui permet d'évacuer des gaz intestinaux à pression réduite et qui comporte un corps (1) oblong pouvant être positionné dans le canal anal et présentant un cylindre (2) étanche à l'eau et aux gaz réalisé en un matériau flexible, l'extrémité du corps (1) laquelle se trouve, en position de fonctionnement, à l'opposé de l'extrémité située du côté du sphincter étant réalisée sous forme d'une pointe (3) en une ou plusieurs parties constituées d'un matériau souple, cette pointe (3) dépassant à son endroit le plus large la largeur du cylindre (2), cette pointe (3) étant humide, et l'intérieur (4) dudit cylindre étant constitué d'un matériau souple à grands pores ressemblant à un filtre, **caractérisé en ce que** la pointe (3) est formée par des fibres naturelles et/ou synthétiques et présente une surface à courbures multiples tout en étant réalisée en plusieurs parties formées par une pluralité de pales qui sont disposées autour de l'axe longitudinal du cylindre (2) et qui sont disposées, le cas échéant, de manière à être comprimées ou à ressembler à des boucles.

2. Dispositif selon la revendication 1, **caractérisé en ce que** le corps (1) comporte au moins un clapet anti-retour (5) ou une membrane, de manière à empêcher que des gaz et des liquides ne pénètrent de l'extérieur dans le canal anal.

3. Dispositif selon la revendication 2, **caractérisé en ce que** le clapet anti-retour (5) ou la membrane est disposé(e) à l'extrémité située, en position de fonctionnement, du côté du sphincter.

4. Dispositif selon la revendication 3, **caractérisé en ce que** le corps (1) présente, à son extrémité située en position de fonctionnement du côté du sphincter, une butée (8) laquelle est située, en position de fonctionnement, à l'extérieur du sphincter et dans laquelle le clapet anti-retour (5) ou la membrane est intégré(e).

5. Dispositif selon l'une des revendications 1 à 4, **caractérisé en ce qu'**un matériau (6) souple est rapporté sur l'extérieur du cylindre (2) afin de permettre une adaptation à l'anatomie individuelle de tout canal anal.

6. Dispositif selon la revendication 5, **caractérisé en ce que** l'ensemble du corps (1) est comprimé en étant vrillé hélicoïdalement.

7. Dispositif selon l'une des revendications 1 à 6, **caractérisé en ce qu'**une cordelette (7) destinée à retirer ledit dispositif du corps est rapportée sur l'extrémité du corps (1) située, en position de fonctionnement, du côté du sphincter.

8. Dispositif selon l'une des revendications 1 à 7, pourvu d'un applicateur, **caractérisé en ce que** ledit applicateur est constitué d'une tige (9) sur laquelle est rapporté un dispositif de déplacement (11), la tige (9) pouvant être positionnée dans l'axe longitudinal du cylindre (2).

9. Dispositif selon l'une des revendications 1 à 7, pourvu d'un applicateur, **caractérisé en ce que** ledit applicateur est constitué d'un cylindre (10) ouvert sur lequel est rapporté le dispositif de déplacement (11), le cylindre (10) ouvert ayant le même axe longitudinal que le corps (1) même, et qu'en position de fonctionnement, le cylindre (10) ouvert entoure le corps (1) de manière à ce qu'au moins la pointe (3) n'est pas recouvert.
